(19) Europäisches Patentamt
European Patent Office
Office européen des brevets

(11) Publication number : 0 229 094 B1

(12) # EUROPEAN PATENT SPECIFICATION

(45) Date of publication of patent specification : **24.04.91 Bulletin 91/17**

(51) Int. Cl.$^5$ : **C12Q 1/04,** G01N 33/569, C12Q 1/06

(21) Application number : **86903473.6**

(22) Date of filing : **21.05.86**

(86) International application number : **PCT/GB86/00285**

(87) International publication number : **WO 86/07094 04.12.86 Gazette 86/26**

(54) **BACTERIAL CELL EXTRACTANTAND METHOD OF EXTRACTING A SUBSTANCE.**

(30) Priority : **22.05.85 GB 8513001**

(43) Date of publication of application : **22.07.87 Bulletin 87/30**

(45) Publication of the grant of the patent : **24.04.91 Bulletin 91/17**

(84) Designated Contracting States : **CH DE FR GB IT LI SE**

(56) References cited :
**EP-A- 0 018 825**
**EP-A- 0 101 398**
**EP-A- 0 108 303**
**FR-A- 2 266 520**
**GB-A- 2 059 990**
**GB-A- 2 116 709**

(56) References cited :
**Experientia, vol. 37, no. 11, Nov. 1981, (Basel, CH), H. Fey et al.: "Experiments for the development of a Salmonella ELISA", see page 7**
**Chemical Abstracts, vol. 84, no. 15, 12 April 1976, (Columbus, Ohio, US), R.M.E. Richards et al.: "Electron microscope study on the effect of benzalkonium chloride and edetate disodium on cell envelope of Pseudomonas aeruginosa", see page 115, abstract no. 100095n**

(73) Proprietor : **University of Wales College of Medicine**
**Heath Park**
**Cardiff CF4 4XN (GB)**

(72) Inventor : **HARBER, John deceased (GB)**

(74) Representative : **James, Michael John Gwynne et al**
**Wynne-Jones, Lainé & James Morgan Arcade Chambers 33, St. Mary Street**
**Cardiff Clamorgan CF1 2AB (GB)**

**EP 0 229 094 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The adenosine triphosphate (ATP) level in bacterial cultures may be used as a measure of bacterial cell numbers, this being an important consideration in the fields of medical microbiology, food and dairy microbiology and environmental microbiology. Extracted ATP is easily measured using the firefly bioluminescence assay, and several medical applications of this methodology have been described including a detection test for bacteriuria, antimicrobial susceptibility tests and assays for antibiotics in clinical specimens. The method also provides a valuable research tool. GB-A-2059990 discloses a technique in which ATP is extracted from a bacterial sample using a mixture of ionic surface agents of the type of ethoxylated amines and quaternary salts of ethoxylated amines. EP-A1-0018825 discloses a technique in which Cetyl-trimethyl-ammonium bromide is used to disrupt the bacterial cell permeability barrier. EP-A1-101398 discloses a technique in which a reagent PICOEX B, which contains a quaternary ammonium salt, is used to lyse the cells of a unicellular organism to release ATP.

Broadly stated, in one aspect, the invention consists in a method of extracting a substance such as ATP (Adenosine Triphosphate) from bacterial cells using a reagent comprising benzalkonium chloride or tetradecyl-trimethyl-ammonium bromide.

According to a preferred feature of the invention the reagent includes a calcium chelator.

The chelator may comprise for example ethylenediamine tetraacetic acid (EDTA) or ethyleneglycol tetraacetic acid (EGTA).

From another aspect the invention consists in a reagent for use, in extracting substances such as ATP (Adenosine Triphosphate) from bacterial cells, comprising tetradecyl-trimethyl-ammonium bromide.

From yet another aspect the invention consists in a reagent for use in extracting substances such as ATP (Adenosine Triphosphate) from bacterial cells, comprising a quaternary ammonium compound and ethyleneglycol tetraacetic acid (EGTA).

In the above method of the invention, the preferred major component of the reagent is benzalkonium chloride. This is a quaternary ammonium compound which consists predominently of $C_{12}H_{25}NC_9H_{13}Cl$ but also contains $C_{14}$ and $C_{16}$ homologues. Alternatively it may be defined as a mixture of alkyldimethyl-benzylammonium chlorides of the general formula

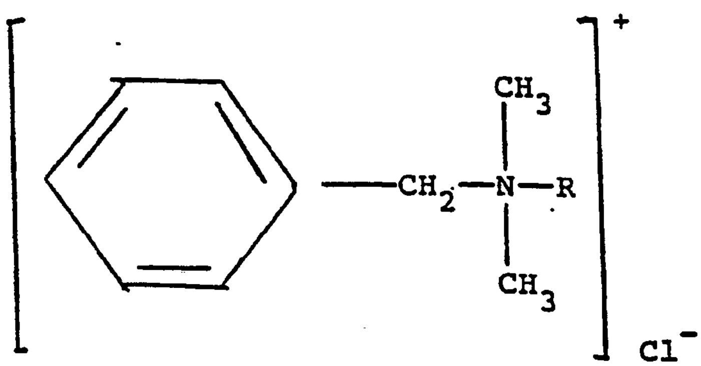

in which R represents a mixture of the alkyls from $C_8H_{17}$ to $C_{18}H_{37}$. A possible alternative is tetradecyl-trimethyl-ammonium bromide. The benzalkonium chloride has been tested at a wide range of concentrations (0.1 to 10.0 mg/ml), with added ethylenediamine tetraacetic acid in the range 1.0 to 50.0 mM, dissolved in water or buffered solutions over the pH range 5.0 to 8.8. The formulation which was found to give the most consistently high efficiency for extracting ATP from a variety of bacterial genera was as follows :

Benzalkonium chloride – 1.0 mg/ml in water plus ethylenediamine tetraacetic acid at 10 mM.

The ethylenediamine tetraacetic acid (EDTA) probably serves two functions. Firstly, it is known to increase the permeability of cell membranes and so probably contributes to the extraction process. Secondly, it inhibits ATP-degrading enzymes so that the released ATP is stable and can therefore be easily measured. The latter appears to be the more important function. Both aspects are dependent on the capacity of EDTA to chelate divalent cations, particularly calcium. The calcium chelator ethyleneglycol tetraacetic acid (EGTA) may be used instead.

The optimum concentrations of the components of the reagent were determined for the extraction of ATP from <u>Escherichia coli</u> growing in nutrient broth no. 2 or human urine, or suspended in phosphate-buffered saline

pH 7.3 (PBS). The reagent was found to be equally effective for ATP extraction over the pH range 5.0 to 8.0, and extraction was achieved within 5-10 seconds in a single, easy step.

The reagent has been compared with other existing extractants for extracting ATP from urinary isolates of E. coli, Klebsiella edwardsii ; Pseudomonas aeruginosa, Proteus mirabilis, Staphylococcus aureus and Streptococcus faecalis. The reagent was as efficient as the other reagents for extracting ATP from bacterial suspension in PBS, and was consistently more efficient than the others for extracting ATP from broth and urine cultures. The commercial reagents, plus the reagent, were all more convenient to use than 2% TCA because extracts prepared with the latter reagent had to be diluted extensively prior to assay to overcome the quenching of light emission produced by this strong acid.

The reagent showed no loss of activity on storage of aqueous solutions at 4°C or 6 months.

The reagent provides a simple, rapid method for extracting bacterial ATP and gives a more consistent extraction efficiency than commercial counterparts when used to extract ATP from bacterial cells under a variety of conditions.

## Claims

1. A method of extracting a substance such as ATP (Adenosine Triphosphate) from bacterial cells using a reagent comprising benzalkonium chloride or tetradecyltrimethyl-ammonium bromide.

2. A method according to Claim 1, wherein said reagent includes a calcium chelator.

3. A method according to Claim 2, wherein the chelator comprises ethylenediamine tetraacetic acid (EDTA) or ethyleneglycol tetraacetic acid (EGTA).

4. A reagent for use in extracting substances such as ATP (Adenosine Triphosphate) from bacterial cells, comprising tetradecyltrimethyl-ammonium bromide.

5. A reagent according to Claim 4, including a calcium chelator.

6. A reagent according to Claim 5, wherein the chelator comprises ethylenediamine tetraacetic acid (EGTA).

7. A reagent for use in extracting substances such as ATP (Adenosine Triphosphate) from bacterial cells, comprising a quaternary ammonium compound and ethyleneglycol tetraacetic acid (EGTA).

8. A reagent according to Claim 7, wherein the quaternary ammonium compound is benzalkonium chloride or tetradecyltrimethyl-ammonium bromide.

## Ansprüche

1. Verfahren zum Extrahieren einer Substanz, wie z.B. ATP (Adenosin-triphosphat) aus Bakterienzellen, bei dem ein Extraktionsmittel verwendet wird, das aus Benzalkoniumchlorid oder Tetradecyltrimethylammoniumbromid besteht.

2. Verfahren nach Anspruch 1, dadurch gekennzeichnet, daß das Extraktionsmittel einen Calcium-Chelator enthält.

3. Verfahren nach Anspruch 2, dadurch gekennzeichnet, daß der Chelator aus Ethylendiamintetraessigsäure (EDTA) oder Ethylenglycoltetraessigsäure (EGTA) besteht.

4. Extraktionsmittel zur Extraktion von Substanzen, wie z.B. ATP (Adenosin-triphosphat) aus Bakterienzellen, dadurch gekennzeichnet, daß es aus Tetradecyltrimetylammoniumbromid besteht.

5. Extraktionsmittel gemäß Anspruch 4, dadurch gekennzeichnet, daß es einen Calcium-Chelator enthält.

6. Extraktionsmittel gemäß Anspruch 5, dadurch gekennzeichnet, daß der Chelator aus Ethylendiamintetraessigsäure (ADTA) besteht.

7. Extraktionsmittel zur Extraktion von Substanzen, wie z.B. ATP (Adenosin-triphosphat) aus Bakterienzellen, dadurch gekennzeichnet, daß es aus einer quaternären Ammoniumverbindung und Ethylenglycoltetraessigsäure (EGTA) besteht.

8. Extraktionsmittel gemäß Anspruch 7, dadurch gekennzeichnet, daß die quaternäre Ammoniumverbindung Benzalkoniumchlorid oder Tetradecyltrimetylammoniumbromid ist.

## Revendications

1. Procédé d'extraction d'une substance telle que l'ATP (Adénosine Triphosphate) à partir de cellules bactériennes, à l'aide d'un réactif comprenant du chlorure de benzalkonium ou du bromure de tétradécyltriméthyl-ammonium.

2. Procédé selon la revendication 1, dans lequel ledit réactif inclut un chélateur du calcium.

3. Procédé selon la revendication 2, dans lequel le chélateur comprend l'acide éthylènediamine tétraacétique (EDTA) ou l'acide éthylèneglycol tétraacétique (EGTA).

4. Réactif destiné à être utilisé dans l'extraction de substances telles que l'ATP (Adénosine Triphosphate) à partir de cellules bactériennes, comprenant du bromure de tétradécyltriméthyl-ammonium.

5. Réactif selon la revendication 4, incluant un chélateur du calcium.

6. Réactif selon la revendication 5, dans lequel le chélateur comprend l'acide éthylènediamine tétraacétique (EDTA).

7. Réactif destiné à être utilisé dans l'extraction de substances telles que l'ATP (Adénosine Triphosphate) à partir de cellules bactériennes, comprenant un composé d'ammonium quaternaire et de l'acide éthylèneglycol tétraacétique (EGTA).

8. Réactif selon la revendication 7, dans lequel le composé d'ammonium quaternaire est le chlorure de benzalkonium ou le bromure de tétradécyltriméthyl-ammonium.